# EUROPEAN PATENT SPECIFICATION

(11) **EP 0 964 645 B1**
(45) Date of publication and mention of the grant of the patent: **25.07.2007**
(21) Application number: 98907596.5
(22) Date of filing: 24.02.1998
(51) Int. Cl.: A61B 17/00, D01F 6/06, A61L 31/04

(54) **METHOD OF PRODUCING A FABRIC**
HERSTELLUNGSVERFAHREN FÜR GEWEBE
PROCEDE DE PREPARATION POUR UN TISSU

(30) Priority: 28.02.1997 US 39208 P
(43) Date of publication of application: 22.12.1999
(73) Proprietor: Sherwood Services AG, 8200 Schaffhausen (CH)
(72) Inventor: DUMICAN, Barry, L., Fairfield, CT 06470 (US); HUTTON, Jeffrey, D., Southbury, CT 06488 (US)
(74) Representative: HOFFMANN EITLE
(86) International application number: PCT/US1998/003535
(87) International publication number: WO 1998/037813

(56) References cited:
- WO-A-97/02789
- US-A- 4 452 245
- US-A- 5 292 328

## Description

### Technical Field

The present invention relates to a process for making a supple monofilament polypropylene mesh. More particularly, the present invention can provide an unexpectedly supple polypropylene monofilament mesh of desirable properties such as superior strength and handling as compared to surgical meshes of equal or greater thickness and weight.

### Background Art

Fabric prosthetic devices for use in surgical procedures are disclosed in U.S. Patent Nos. 2,671,44; 3,054,406; 3,124,136; 3,276,448; 4,347,847; 4,452,245; 4,633,873; 4,655,221; 4,838,884; 5,002,551; 5,116,357; 4,147,374; 5,292,328; 5,368,602; 5,297,331; 5,425,740 and 5,433,996. Known types of mesh or fabric devices range from meshes constructed from resins, such as those disclosed in U.S. Patent Nos. 2,671,444 and 3,124,136, to surgical meshes constructed of yarns or multifilament threads, such as that disclosed in U.S. Patent No. 5,292,328. Most of the prior art surgical mesh or fabric prosthetic devices were designed for use in a wide variety of internal surgical procedures, such as for example pressure encapsulation and repair of traumatically damaged organs such as the spleen, liver or kidney, or the repair of abdominal or chest wall defects, hernias, urinary tract defects or the like.

It is believed that although nonabsorbable prosthetic meshes constructed from polypropylene monofilament induces a good fibroblastic response to ensure prompt fixation and integration with tissue at the surgical repair site, it has a poor hand and is too stiff and cumbersome for some surgical procedures. A need thereby exists for a polypropylene monofilament mesh which induces good fibroblastic response to ensure prompt fixation and integration with tissue at the surgical repair site which it is known to do and is supple enough to be easily manipulated for use in any surgical procedure routinely requiring such a device including giant prosthetic reinforcement of the visceral sac.

WO 97/02789 discloses an implantable dual bar warp knit, hexagonal mesh, suitable for repair of a tissue or muscle wall defect. This prosthetic repair material may be formed from polypropylene monofilament threads.

### Disclosure of Invention

The fabric prosthesis is generally comprises of a supple, flexible mesh material produced by the process of the present invention which is non-ravelling when cut. The fabric is made of tricot knit monofilament polypropylene which is thinner and lighter in weight than current commercially available products while possessing superior strength and suppleness. Prior to the present invention, a "supple" monofilament polypropylene mesh was not considered possible as stated in U.S. Patent No. 5,292,328. However, the surprising suppleness of the subject fabric, obtained through the unique process of the present invention allows for greater control, conformability and suturability of the mesh in the variety of surgical procedures which require such devices.

The process for producing the subject supple monofilament polypropylene mesh eliminates the filament annealing process step, allows for the doubling of the typical filament production line speed without compromising quality and provides for the use of a water soluble spin finish each of which increases the rate of production and decreases associated costs.

Accordingly, it is an object of the present invention to provide a process which yields a surgical prosthetic device which is of lower weight and thickness than currently marketed devices with increased strength and suppleness for various internal surgical uses such as those noted above.

It is a further object of the present invention to provide a process for making the surgical prosthetic device of the present invention which requires-less time to produce and is more economically manufactured.

Still other objects, features and advantages of the present invention shall become apparent in view of the following detailed description when considered in connection with the accompanying example.

### Mode(s) for Carrying Out the Invention

The surgical mesh is generally comprised of a thin, flexible monofilament polypropylene mesh material having any one of a variety of different configurations depending on the intended application of the mesh in a surgical procedure. The dimensions of the mesh are only limited by the practical size for its intended surgical use. Dimensions within the range of 13 x 76 mm (1/2 x 3 inches) to 31 x 41 cm (12 x 16 inches) with a thickness of .3 to .8 mm but preferably about 6 mm has been found desirable for surgical handling ease. A weight within the range of 0.068 to 0.14 kg/m² (2.0 to 4.0 ounces) but most preferably 1 kg/m² (3.0 ounces per square yard) is desired for adequate strength and manageability. A drawing which describes the shape and/or geometric configuration of the subject mesh or fabric is not necessary for understanding the present invention. Any person skilled in the art of mesh or fabric will know how to manufacture and how to use the subject invention by reading this specification, generally, and the example, specifically. The surgical mesh of the present invention comprises tricot knitted polypropylene monofilament indicated for use in the repair of soft tissue defects, e.g., hernia repair, chest wall reconstruction or like procedures as noted briefly above requiring the addition of a reinforcing or bridging material.

The subject surgical mesh is produced from polypropylene monofilament fibers approximately 0.08 to 0.20 mm (0.003 to 0.008 inches) in diameter but preferably approximately 0.152 mm (0.006 inches) in diameter to achieve the desired strength, i.e., a ball burst load of 60 to 75 kg.

In general, the polypropylene monofilament fibers used in the production of the subject surgical mesh are prepared as known to those skilled in the art and disclosed in U.S. Patent Nos. 3,630,205, 4,911,165 and 5,217,485. More specifically, the polypropylene monofilament fibers used in the present mesh are produced by a process comprising the steps of:
a) extruding polypropylene through one or more orifices;
b) quenching the polypropylene rapidly in a water bath to produce one or more filaments;
c) drawing the filament at least once;
d) applying a water soluble spin finish; and
e) collecting on a spool.

In carrying out the process of producing the mesh of the present invention, the preferred polypropylene resin for filament production is a surgical suture grade resin having an isotactic index of 97 (97% isotactic) or above but preferably having an isotactic index of 98 or 99 (98% or 99% isotactic), and a melt flow rate of from about 3.5 to 4.5 gms/10 min. but preferably 4 gms/10 min. to achieve a desirable production viscosity. The polypropylene resin extruded as a filament has a denier between 100 and 200 but preferably 150 to achieve desirable strength and handling characteristics.

The described surgical suture grade resin is extruded at a flat temperature of approximately 225°C through at least one orifice and rapidly quenched in a water bath of approximately 25°C. The filament produced is drawn at least once but most preferably twice to align the polymer molecules to improve physical characteristics and enhance the overall quality thereof. The first drawing is carried out at a temperature of approximately 120°C at a draw ratio of approximately 5.93:1. The second drawing is carried out at a temperature of approximately 130°C at a draw ratio of approximately 1.43:1. It was found that the two draws as described herein allowed the output line speed to be increased from 56 m/min (185 feet per minute) to 113 m/min (370 feet per minute) without void formation or increased frays.

A water soluble spin finish is then applied to the extruded and drawn filament. Suitable spin finish lubricants may be selected from the group consisting of water-soluble low molecular weight, i.e., approximately 200 to 10,000 molecular weight, C₂₋₇ polyalkyl glycol coatings. The preferred spin finish is poly(ethylene glycol) 400 monopelargonate lubricant which is easily removed in water and has favorable toxicology. Another water soluble lubricant which can be implemented in the present invention is Lurol" manufactured by Goulston Technologies but the same is not preferred. The selected spin finish is preferably applied to the polypropylene filament between the last draw godget and the spool winder heads which are used to wind the filament onto collection spools. One method of applying the finish lubricant includes the use of a cellulose sponge with half depth slits therein wetted with a 50:50 volume:volume solution of the finish lubricant in water. The sponge is wetted with the solution by pouring the same over the sponge. The filaments are then passed through the sponge slits to lubricate the filaments.

An alternate method of lubricating the filament includes the use of a 15 cm (6 inch) aluminum cylinder combined with a drive system to cause the cylinder to rotate about its axis through a finish reservoir filled with lubricant. The reservoir is filled with 100% finish which coats the cylinder upon rotation thereof through the reservoir. Each filament is passed over the coated cylinder surface at about a 5 or 10 percent contact angle. A cylinder roll speed of approximately 1 revolution per minute has been found to provide sufficient lubricant on the fiber for ease in the subsequent knitting thereof.

Following the lubrication of the filament, the mesh can be fabricated from the non-annealed filament employing known and conventional knitting apparatus and techniques. Preferably, the subject mesh is manufactured using a tricot knitting machine with single bar action and 5.5 needles per cm (14 needles per inch). Optionally, an extra filament of monofilament polypropylene can be used in the fabric selvages in accordance with methods known to those skilled the art to simplify the knitting process and to improve the selvage appearance.

Once the polypropylene monofilament has been knitted into the desired mesh, the spin finish is removed using water. A standard washing machine may be used to wash the mesh and optionally a detergent and/or defoamer may be used. The wash water temperature is preferably approximately 55°C (130°F) with a cold water rinse to minimize shrinkage.

The mesh after washed and air dried is heat set at 145°C for 10 minutes to eliminate curling and set the filaments. Increasing the temperature to 150°C yielded decreases in the physical properties of the mesh and lower temperatures resulted in filaments being "set" to a lesser degree and a coarser "hand" or feel to the fabric.

The heat set mesh may be cut to the desired dimensions using any suitable cutting technique such as but not limited to hot knife, laser or ultrasonic knife. Preferably, the mesh is cut using a hot knife for economical considerations to fuse the edges and thereby trap any loose cut ends.

The mesh may be packaged using any suitable means and materials known in the art. The preferred packaging is a polyethylene terphthalate film fused to non-woven polyethylene terphthate web coated with polyethylene resin to form a pouch with an optional interior paper folder. The folder is advantageous for positioning the mesh for easy extraction during a surgical procedure.

Any suitable sterilization technique known to those skilled in the art may be used such as steam autoclaving, pre-vacuum steam autoclaving or ethylene oxide. However, ethylene oxide sterilization is preferred for ease and effectiveness.

It can also be advantageous to provide the surgical mesh with a clearly visible color pattern such as for example in the form of a grid of two differently colored filaments. Such a pattern can facilitate proper orientation of the mesh at the surgical repair site.

The process for producing the polypropylene monofilament mesh is further described in the following example.

### EXAMPLE:

The conditions of melt spinning the polypropylene monofilament fiber for the subject surgical mesh and the specific properties of the mesh are set forth in the following Tables 1 through 4:

**TABLE 1: Polypropylene Monofilament Manufacturing Conditions**

| Operating Conditions | | | |
|---|---|---|---|
| Component | Conditions | Component | Conditions |
| Extruder Barrel Temp., °C | 225°C | Spinneret Pressure, psi | 1000-2500 |
| Block Temp., °C | 225°C | First Draw Oven, °C | 120°C |
| Clamp Temp., °C | 225°C | Draw Ratio | 5.93 |
| Spinneret Temp., °C | 225°C | Second Draw Oven, °C | 130°C |
| No. of Spinneret Orifices | 8 | Draw Ratio | 1.43 |
| Diameter of Spinneret | .02 inch | | |
| Orifices, inch | ≡ 0.5 mm | | |

**TABLE 2: Filament Properties**

| | |
|---|---|
| Denier | 150 |
| Straight Pull Strength, kg | 1.2 - 2.0 |
| Breaking Elongation, percent | 18-27 |
| Young's Modulus, kpsi | 740-950 ≡ 5000 MPa |

**TABLE 3: Knitting Conditions**

| | |
|---|---|
| Knitting Machine: | 14 gauge 1-bar tricot Knitter |
| Length of Filament/Rack, inches | 79-86 (≡ 2·0-2-2 m) |
| Length of Mesh/Rack, inches | 12-14 (≡ 30-35 cm) |

**TABLE 4: Monofilament Polypropylene Surgical Mesh Properties**

| | |
|---|---|
| weight, oz./sq. yd. | 2.88 (≡ 0.097 kg/m²) |
| Thickness, mm | .59 |
| Ball Burst Load, kg | 70.9 |
| Ball Burst Displacement, inches | .48 (≡ 12 mm) |

The unique process of the present invention described in detail herein unexpectedly produces a polypropylene monofilament surgical mesh of improved physical properties, such as increased suppleness and strength. The mesh is characterized by having a compliancy similar to that of a polyester mesh or multifilament mesh so as to be suitable for use as a prosthetic device in hernioplasty procedures and in particular suitable as a prosthetic device for giant prosthetic reinforcement of the visceral sac. invention may be constructed without departing from the scope of the underlying inventive concept and that the same is not limited to the particular forms herein described except insofar as indicated by the scope of the appended claims.

## Claims

1. A method of producing a supple monofilament polypropylene mesh comprising:
extruding polypropylene;
quenching the extruded polypropylene in a water bath to form a filament;
drawing the filament at least once;
coating the filament with a water soluble lubricant;
knitting the filament into a mesh; and
heat setting the mesh.

2. A method according to Claim 1, wherein said water soluble lubricant comprises a low molecular weight C₂₋₇ polyalkyl glycol.

3. A method according to Claim 1, wherein said polypropylene is extruded at approximately 225°C.

4. A method according to Claim 1, wherein the filament is drawn at a temperature of 120°C at a ratio of approximately 5.93:1, and then drawn at a temperature of 130°C at a ratio of approximately 1.43:1.

5. A method according to Claim 1, wherein said water soluble lubricant is poly(ethyleneglycol) 400 monopelargonate or Lurol^{™}.

6. A method according to Claim 1, wherein said mesh is a tricot knit which is non-raveling when cut.

7. A method according to Claim 1, wherein said mesh is heat set at 145°C for 10 minutes.

8. A method according to Claim 1, wherein said filament is produced at a production line speed of approximately 1.9 m/sec (380 feet per minute).

9. A method according to Claim 1, further comprising packaging and sterilizing the mesh for surgical use.

10. A method according to Claim 1, wherein said mesh is packaged in a polymeric envelope and sterilized using ethylene oxide.

11. A method according to Claim 1, further comprising removing the lubricant using water prior to heat setting.

12. A method according to any preceding claim comprising the steps of:
extruding surgical suture grade polypropylene **characterized by** an isotactic index of approximately 97 to 99 and a melt flow rate of approximately 3.5 to 4.5 gms/10 min. at a flat temperature of approximately 225°C;
quenching the extruded polypropylene in a water bath of approximately 25°C to form a filament of from 100 to 200 denier;
drawing the filament at 120°C at a ratio of approximately 5.93:1;
drawing the filament at 130°C at a ratio of approximately 1.43:1;
coating the filament with a water soluble lubricant;
knitting the filament into a mesh;
washing the mesh to remove the lubricant; and heat setting the mesh at 145°C for 10 minutes.

## Patentansprüche

1. Verfahren zur Herstellung eines biegsamen Monofilament-Polypropylen-Netzes, das die folgenden Schritte umfaßt:
Extrudieren von Polypropylen;
Quenchen des extrudierten Polypropylens in einem Wasserbad zur Bildung eines Filaments;
wenigstens einmaliges Strecken des Filaments;
Beschichten des Filaments mit einem wasserlöslichen Schmiermittel;
Stricken des Filaments zu einem Netz; und
Thermofixierung des Netzes.

2. Verfahren gemäß Anspruch 1, worin das wasserlösliche Schmiermittel ein C₂₋₇-Polyalkylglycol mit geringem Molekulargewicht umfaßt.

3. Verfahren gemäß Anspruch 1, worin das Polypropylen bei ungefähr 225°C extrudiert wird.

4. Verfahren gemäß Anspruch 1, worin das Filament bei einer Temperatur von 120°C und mit einem Verhältnis von ungefähr 5,93:1 gestreckt wird und anschließend bei einer Temperatur von 130°C und mit einem Verhältnis von ungefähr 1,43:1 gestreckt wird.

5. Verfahren gemäß Anspruch 1, worin das wasserlösliche Schmiermittel Poly(ethylenglycol) 400 Monopelargonat oder Lurol^{™} ist.

6. Verfahren gemäß Anspruch 1, worin das Netz eine Trikot-Maschenware ist, das sich nicht auftrennt, wenn es geschnitten wird.

7. Verfahren gemäß Anspruch 1, worin das Netz bei 145°C für 10 Minuten thermofixiert wird.

8. Verfahren gemäß Anspruch 1, worin das Filament mit einer Produktionsliniengeschwindigkeit von ungefähr 1,9 m/s (380 Fuß pro Minute) hergestellt wird.

9. Verfahren gemäß Anspruch 1, welches zusätzlich das Verpacken und Sterilisieren des Netzes für die chirurgische Verwendung umfaßt.

10. Verfahren gemäß Anspruch 1, worin das Netz in einem Polymerumschlag verpackt und unter Verwendung von Ethylenoxid sterilisiert wird.

11. Verfahren gemäß Anspruch 1, welches zusätzlich das Entfernen des Schmiermittels unter Verwendung von Wasser vor der Thermofixierung umfaßt.

12. Verfahren gemäß einem der vorhergehenden Ansprüche, das die folgenden Schritte umfaßt:
Extrudieren von Polypropylen von chirurgischer Wundnahtqualität, **dadurch gekennzeichnet, daß** es einen isotaktischen Index von ungefähr 97 bis 99 und einen Schmelzdurchfluß von ungefähr 3,5 bis 4,5 g/10 min bei einer ebenen Temperatur von ungefähr 225°C aufweist; Quenchen des extrudierten Polypropylens in einem Wasserbad bei ungefähr 25°C zur Bildung eines Filaments mit 100 bis 200 Denier;
Strecken des Filaments bei 120°C und mit einen Verhältnis von ungefähr 5,93:1;
Strecken des Filaments bei 130°C und mit einem Verhältnis von ungefähr 1,43:1;
Beschichten des Filaments mit einem wasserlöslichen Schmiermittel;
Stricken des Filaments zu einem Netz;
Waschen des Netzes zur Entfernung des Schmiermittels; und
Thermofixierung des Netzes bei 145°C für 10 Minuten.

## Revendications

1. Méthode de production d'une maille de polypropylène monofilamentaire souple comprenant:
l'extrusion du polypropylène;
la trempe du polypropylène extrudé dans un bain d'eau pour former un filament;
l'étirage du filament au moins une fois;
l'enrobage du filament au moyen d'un lubrifiant soluble dans l'eau;
le tricotage du filament en une maille; et
le durcissement à la chaleur de la maille.

2. Méthode selon la revendication 1, où ledit lubrifiant soluble dans l'eau comprend un polyalkyl glycol C₂₋₇ de faible poids moléculaire.

3. Méthode selon la revendication 1, où ledit polypropylène est extrudé aux environs de 225°C.

4. Méthode selon la revendication 1; où le filament est étiré à une température de 120°C à un rapport d'environ 5,93:1, puis est étiré à une température de 130°C à un rapport d'environ 1,43:1.

5. Méthode selon la revendication 1, où ledit lubrifiant soluble dans l'eau est du monopélargonate de poly(éthylèneglycol) 400 ou Lurol^{™}.

6. Méthode selon la revendication 1, où ladite maille est un tricot qui ne se défile pas quand il est coupé.

7. Méthode selon la revendication 1, où ladite maille est durcie à la chaleur à 145°C pendant 10 minutes

8. Méthode selon la revendication 1, où ledit filament est produit à une vitesse de production en ligne d'environ 1,9 m/s (380 pieds par minute).

9. Méthode selon la revendication 1, comprenant de plus l'emballage et la stérilisation de la maille pour un usage chirurgical.

10. Méthode selon la revendication 1, où ladite maille est emballée dans une enveloppe polymérique et stérilisée en utilisant de l'oxyde d'éthylène.

11. Méthode selon la revendication 1, comprenant de plus l'élimination du lubrifiant en utilisant de l'eau avant durcissement à la chaleur.

12. Méthode selon toute revendication précédente comprenant les étapes de:
extruder du polypropylène de qualité chirurgicale **caractérisé par** un indice isotactique d'environ 97 à 99 et une vitesse d'écoulement en phase fondue d'environ 3,5 à 4,5 g/10 mn. à une température à plat d'environ 225°C;
tremper le polypropylène extrudé dans un bain d'eau à environ 25°C pour former un filament d'environ 100 à 200 deniers;
étirer le filament à 120°C à un rapport d'environ 5,93:1;
étirer le filament à 130°C à un rapport d'environ 1, 43:1;
enrober le filament d'un lubrifiant soluble dans l'eau;
tricoter le filament en une maille;
laver la maille pour éliminer le lubrifiant; et
durcir à la chaleur la maille à 145°C pendant 10 minutes.
